# FASCICULE DE BREVET EUROPEEN

(11) **EP 0 761 203 B1**
(45) Date de publication et mention de la délivrance du brevet: **10.12.1997**
(21) Numéro de dépôt: 96401633.1
(22) Date de dépôt: 22.07.1996
(51) Int. Cl.: A61K 7/48

(54) **Utilisation de l'acide cystéique ou homocystéique pour favoriser la desquamation de la peau ou stimuler le renouvellement épidermique**
Verwendung von Cysteinsäure oder Homocystein zur Förderung der schuppende Haut oder Stimulierung der Epidermiserneuerung
Use of cysteine acid homocysteine for enhancing the desquamation of the skin or to stimulate the renewal of the epidermis

(30) Priorité: 07.09.1995 FR 9510483
(43) Date de publication de la demande: 12.03.1997
(73) Titulaire: L'OREAL, 75008 Paris (FR)
(72) Inventeur: Maignan, Jean, 93290 Tremblay (FR)
(74) Mandataire: Andral, Christophe André Louis

(56) Documents cités:
- EP-A- 0 368 758
- US-A- 4 053 630
- US-A- 4 224 339
- DATABASE WPI Week 8929 Derwent Publications Ltd., London, GB; AN 89-210007 XP002005305 & JP-A-01 146 816 (LION CORPORATION) , 8 Juin 1989

## Description

L'invention se rapporte à l'utilisation d'acides sulfoniques particuliers dans ou pour la fabrication d'une composition cosmétique et/ou dermatologique pour favoriser la desquamation de la peau et/ou lutter contre le vieillissement intrinsèque et extrinsèque de la peau. Elle se rapporte aussi à un procédé de traitement non thérapeutique de la peau en vue de desquamer la peau ainsi qu'à un procédé de traitement du vieillissement cutané.

Le vieillissement cutané résultant d'effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement 〈〈 normal 〉〉 lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4 603 146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A- 413 528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les bêta-hydroxy-acides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau, consistant en une desquamation, c'est-à-dire l'élimination des cellules 〈〈 mortes 〉〉 situées à la surface du *stratum corneum*. Cette propriété desquamante est aussi appelée, souvent à tort, propriété kératolytique. Mais ces composés présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

La demanderesse a trouvé de manière inattendue que l'application topique d'acide cystéique ou homocystéique ou d'un de leurs dérivés permettait de desquamer la peau ainsi que de stimuler le renouvellement cellulaire épidermique et la réparation épidermique.

Certes, les acides cystéique et homocystéique sont déjà connues dans le domaine pharmaceutique pour le traitement de la peau sèche, des verrues, des kératoses actiniques ou non, de l'acné, des ichtyoses et des hyperkératoses palmaires et plantaires (voir le document US-A-4 224 339). Cependant personne jusqu'à ce jour n'a envisagé ni suggéré d'utiliser ces acides pour la desquamation de la peau, la stimulation du renouvellement épidermique et le traitement du vieillissement cutané.

Aussi, la présente invention a pour objet l'utilisation d'au moins un acide sulfonique à l'état libre ou au moins partiellement neutralisé, dans ou pour la fabrication d'une composition cosmétique ou dermatologique pour favoriser la desquamation de la peau et/ou stimuler le renouvellement épidermique, cet acide répondant à la formule (I) suivante : 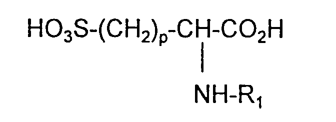 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle.

La desquamation de la peau est associée à une amélioration clinique de la qualité de la peau qui devient plus éclatante, moins ridée et d'une façon générale plus jeune. De plus, l'utilisation des acides sulfoniques ci-dessus permet de traiter les imperfections de la peau telles que les taches, les dyschromies cutanées, les dermites, les lentigos actiniques, les cicatrices et les pigmentations cicatricielles.

Aussi, l'invention a encore pour objet l'utilisation d'au moins un acide sulfonique à l'état libre ou au moins partiellement neutralisé, dans ou pour la fabrication d'une composition cosmétique ou dermatologique comme agent antivieillissement, notamment pour lutter contre les rides et/ou les ridules et/ou les taches actiniques et/ou les dyschromies cutanées et/ou les dermites et/ou les cicatrices.

La demanderesse a en particulier constaté, sans que cela soit complètement expliqué, que ces composés ont une action antivieillissement au moins aussi efficace que les composés de l'art antérieur qui sont tous des acides carboxyliques et que cette action est plus douce dans la mesure où aucune irritation n'est ressentie et aucune rougeur n'est observée lors de l'application sur la peau d'une composition cosmétique ou dermatologique les contenant.

En particulier, les acides de l'invention sont les acides cystéique et homocystéique. Ces deux acides ont l'avantage d'être disponibles sur le marché. Les autres acides de l'invention peuvent être obtenus suivant des méthodes connues et par exemple en faisant réagir sur les produits de formule (I) avec R₁ représentant H, une forme activée d'un acide R₂-COOH qui peut être un produit anhydre, un anhydride mixte (obtenu à partir d'un chloroformiate) ou un chlorure d'acide (ClCOR₂), en maintenant le pH à une valeur comprise dans la gamme de 7 à 9 (réaction de Schotten-Baumann). En fin de réaction, le produit obtenu est précipité à pH acide (pH = 1) ou extrait selon les techniques usuelles.

Toute ou partie des acides de l'invention peut être neutralisée, pour former un sel, notamment par la soude, la potasse, l'ammoniaque ou encore la mono-éthanolamine, la triéthanolamine et l'isopropanolamine.

Dans les compositions selon l'invention, l'acide sulfonique ou le mélange d'acides sulfoniques peut être utilisé en une quantité allant de 0,2 à 20 % en poids par rapport au poids total de la composition et en particulier en une quantité allant de 0,5 à 10 % et mieux de 0,5 à 5 % en poids par rapport au poids total de la composition.

Les acides de l'invention peuvent être associés à d'autres actifs connus pour leurs propriétés desquamantes, comme les hydroxy-acides, les α- ou β-céto-acides, les rétinoïdes. Une telle association permet de diminuer la concentration active de ces derniers du fait des effets additifs. On peut ainsi obtenir une composition moins irritante et moins toxique ainsi qu'une composition plus efficace que celles de l'art antérieur n'utilisant que ces actifs.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Ces hydroxyacides sont notamment les acides glycolique, lactique, malique, tartrique, citrique et de manière générale les acides de fruits, les acides hydroxy-2 alcanoïque, mandélique, salicylique, ainsi que leurs dérivés alkylés ou acylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels, ou encore le rétinal.

A titre d'exemple, les hydroxyacides, les céto-acides et les rétinoïdes peuvent être utilisés dans les compositions selon l'invention en une quantité représentant de 0,1 à 5 % en poids du poids total de la composition et mieux de 0,5 à 3 %.

En vue de lutter efficacement contre le photovieillissement, il est en outre possible d'ajouter à la composition de l'invention un ou plusieurs filtres solaires complémentaires, actifs dans l'UVA et/ou l'UVB, hydrophiles ou lipophiles.

Un test *in vitro* d'efficacité de la desquamation a été réalisé sur kératinocytes en utilisant de l'acide n-octanoyl-5-salicylique (composé 1), de l'acide cystéique (composé 2), de l'ester bis-(1-éthoxycarbonyl-éthyl)- de l'acide nonanedioique (composé 3), de l'acide 2-acétoxy-5-octanoyl-benzoique (composé 4) et de l'acide 5-oxothiomorpholine-3-carboxylique (composé 5).

Le principe du test repose sur le fait que la desquamation induit la libération de cornéocytes. Le pouvoir de desquamation du produit testé va être d'autant plus grand que le nombre de cornéocytes libérés sera important.

Le protocole du test a été le suivant : à partir de biopsies de peau, on a obtenu, par séparation de l'épiderme, des kératinocytes que l'on a dissociés par action enzymatique à la trypsine et mis en culture à la concentration de 2.10⁻⁵ cellules/ml. La croissance et la différenciation des kératinocytes ont été obtenues par culture durant 10 à 20 jours en milieu spécifique.

Puis, après élimination du milieu de culture, on a ajouté le produit à tester et évalué l'activité du produit. Pour ce faire, on a réalisé deux prélèvements à T₀ et T₆₀, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et révèle donc exclusivement la présence des kératinocytes.

L'indice de détachement cellulaire est déterminé par la différence entre T₆₀ et T₀.

La même mesure a été réalisée pour un témoin ne contenant pas de produit à tester car l'expérience produit inévitablement la libération de cornéocytes, même en l'absence d'actif. La variation du témoin a fixé arbitrairement la norme de 100 %.

Les résultats sont rassemblés dans le tableau ci-dessous :

| Témoin | Composé 1 | Composé 2 | Composé 3 | Composé 4 | Composé 5 |
|---|---|---|---|---|---|
| 0 % | 106 % | 206 % | 59 % | inactif | inactif |

Ces résultats montrent clairement que l'acide cystéique, à concentration égale à celle de l'acide n-octanoyl-5-salicylique connu comme étant un actif desquamant puissant, est beaucoup plus actif que celui-ci, et que les autres composés ont des activités plus faibles que celle de l'acide cystéique.

L'invention a encore pour objet un procédé de traitement cosmétique ou dermatologique de la peau destiné à la desquamation de la peau consistant à appliquer sur la peau une composition contenant au moins un acide sulfonique à l'état libre ou partiellement neutralisé de formule (I), dans un milieu cosmétiquement et/ou dermatologiquement acceptable.

L'invention a aussi pour objet un procédé de traitement cosmétique ou dermatologique du vieillissement de la peau, consistant à appliquer sur la peau une composition contenant au moins un acide sulfonique tel que défini ci-dessus, dans un milieu cosmétiquement et/ou dermatologiquement acceptable.

La composition de l'invention contient un milieu cosmétiquement ou dermatologiquement acceptable, c'est-à-dire un milieu compatible avec la peau, les ongles, les muqueuses, les tissus et les cheveux. La composition contenant l'acide sulfonique peut être appliquée par voie topique sur le visage, le cou, les cheveux, les muqueuses et les ongles ou toute autre zone cutanée du corps.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarate de sorbitane vendus respectivement sous les dénominations commerciales Tween 60 et Span 60 par la Société ICI. On peut y ajouter des coémulsionnants tels que le PPG-3 myristyl éther vendu sous la dénomination commerciale Emcol 249-3K par la société Witco.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

On peut, entre autre, associer les acides sulfoniques à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.

Le procédé de traitement cosmétique ou dermatologique de l'invention peuvent être mis en oeuvre notamment en appliquant les compositions hygiéniques, cosmétiques ou dermatologiques telles que définies ci-dessus, selon la technique d'utilisation habituelle de ces compositions. Par exemple : application de crèmes, de gels, de sérums, de pommades, de lotions, de laits sur la peau, le cuir chevelu, les ongles et/ou les muqueuses.

Les exemples suivants illustrent l'invention. Dans ces exemples, les proportions indiquées sont des pourcentages en poids.

### Exemple 1 : Emulsion H/E

| *Phase A :* | |
|---|---|
| - Acide cystéique | 2,5 |
| - Huile d'amande douce | 14,5 |
| - Huile de karité | 7,0 |
| - PPG-3 myristyl éther (EMCOL 249-3K) | 5,0 |
| - Conservateur (propylparabène) | 0,1 |
| - Polysorbate 60 (TWEEN 60) | 2,5 |
| - Stéarate de sorbitane (SPAN 60) | 2,5 |

| *Phase B :* | |
|---|---|
| - Cyclométhicone | 4,0 |
| - Gomme de xanthane | 0,2 |
| - Polymère carboxyvinylique | 0,5 |

| *Phase C :* | |
|---|---|
| - Triéthanolamine (neutralisant) | 0,5 |
| - Eau | 2,0 |

| *Phase D :* | |
|---|---|
| - Conservateur (méthylparabène) | 0,2 |
| - Glycérine | 5,0 |
| - Eau qsp | 100 |

### Mode opératoire :

On fait fondre les constituants de la phase A à 85° C, puis on refroidit la phase A à 70° C et on y introduit les phases B puis C et D sous agitation. On refroidit jusqu'à la température ambiante. On obtient une crème de jour qui provoque la desquamation de la peau et confère ainsi à celle-ci un aspect plus lisse et plus jeune qu'avant le traitement.

### Exemple 2 : Gel

| | |
|---|---|
| - Acide homocystéique | 5,0 |
| - Hydroxypropylcellulose (Klucel H de la société Hercules) | 1,0 |
| - Antioxydant | 0,05 |
| - Isopropanol | 40,0 |
| - Conservateur | 0,3 |
| - Eau qsp | 100 |

On obtient un gel qui, en application régulière, permet d'estomper les taches de la peau par desquamation.

### Exemple 3 : Solution pour application dermatologique

| | |
|---|---|
| - Acide cystéique | 5,00 |
| - Antioxydant | 0,05 |
| - Alcool éthylique | 10,00 |
| - Conservateur | 0,30 |
| - Eau qsp | 100 |

L'application sous contrôle dermatologique de cette solution permet d'obtenir une desquamation profonde de la couche cornée et, ainsi, la mise en jeu d'un processus de réparation épidermique, ayant comme effet thérapeutique final un effacement des taches et dyschromies, un estompement des rides et ridules et une amélioration de l'état clinique de la peau, dont l'aspect devient celui d'une peau plus jeune.

Cette application se fait à raison d'une à trois séances hebdomadaires pendant 4 à 6 semaines.

## Revendications

1. Utilisation d'au moins un acide sulfonique à l'état libre ou au moins partiellement neutralisé, dans ou pour la fabrication d'une composition cosmétique ou dermatologique pour favoriser la desquamation de la peau et/ou stimuler le renouvellement épidermique, cet acide répondant à la formule (I) suivante : 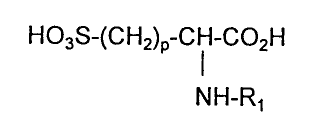 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle.

2. Utilisation d'au moins un acide sulfonique à l'état libre ou au moins partiellement neutralisé, dans ou pour la fabrication d'une composition cosmétique ou dermatologique, pour lutter contre le vieillissement cutané, cet acide répondant à la formule (I) suivante : 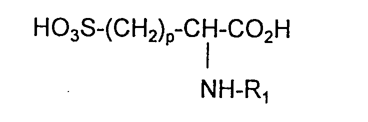 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle..

3. Utilisation d'au moins un acide sulfonique à l'état libre ou au moins partiellement neutralisé, dans ou pour la fabrication d'une composition dermatologique pour lutter contre les rides et/ou les ridules et/ou les taches actiniques et/ou les dyschromies cutanées et/ou les cicatrices et/ou les dermites, cet acide répondant à la formule (I) suivante : 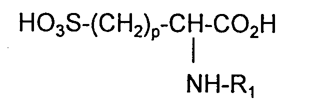 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle.

4. Utilisation selon l'une des revendications 1 à 3, caractérisée en ce que l'acide sulfonique est l'acide cystéique ou l'acide homocystéique.

5. Utilisation selon l'une des revendications précédentes, caractérisée en ce que l'acide sulfonique est utilisé en une quantité allant de 0,2 à 20 % et de préférence de 0,5 à 5 % en poids par rapport au poids total de la composition.

6. Utilisation selon l'une des revendications précédentes, caractérisée en ce que la composition comprend, en outre, au moins un actif choisi parmi les α- ou β-hydroxy-acides, les α- ou β-céto-acides et les rétinoïdes.

7. Utilisation selon l'une des revendications précédentes, caractérisée en ce que la composition comprend, en outre, au moins un actif choisi parmi les acides glycolique, lactique, malique, tartrique, citrique, hydroxy-2 alcanoïque, mandélique, salicylique, l'acide n-octanoyl-5-salicylique.

8. Utilisation selon la revendication 6 ou 7, caractérisée en ce que l'actif est présent en une quantité allant de 0,1 à 5 % en poids par rapport au poids total de la composition.

9. Utilisation selon l'une des revendications précédentes, caractérisée en ce que la composition contient, en outre, au moins un adjuvant choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles.

10. Utilisation selon l'une des revendications précédentes, caractérisée en ce que la composition est une solution aqueuse, huileuse ou hydroalcoolique, une émulsion eau-dans-huile ou huile-dans-eau, une microémulsion, un gel aqueux ou anhydre, un sérum, une dispersion de vésicules, de microcapsules ou de microparticules.

11. Procédé de traitement cosmétique de la peau destiné à la desquamation de la peau consistant à appliquer sur la peau une composition contenant au moins un acide sulfonique à l'état libre ou partiellement neutralisé de formule (I) suivante : 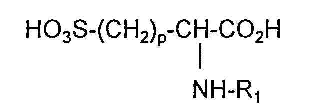 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle.

12. Procédé de traitement cosmétique du vieillissement de la peau consistant à appliquer sur la peau une composition contenant au moins un acide sulfonique à l'état libre ou partiellement neutralisé de formule (I) suivante : 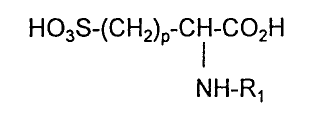 avec p vallant 1 ou 2 et R₁ représentant un atome d'hydrogène ou un reste acyle de structure -COR₂ où R₂ est un groupement alkyle ou alcényle en C₁ à C₁₉, linéaire ou ramifié, substitué ou non par au moins une fonction hydroxyle.

## Claims

1. Use of at least one sulphonic acid, in the free state or at least partially neutralized, in or for the manufacture of a cosmetic or dermatological composition for promoting desquamation of the skin and/or for stimulating epidermal renewal, this acid corresponding to the following formula (I): 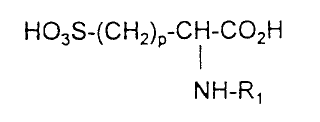 with p having the value 1 or 2 and R₁ representing a hydrogen atom or an acyl residue of structure -COR₂ where R₂ is a linear or branched C₁ to C₁₉ alkyl or alkenyl group which is unsubstituted or substituted by at least one hydroxyl functional group.

2. Use of at least one sulphonic acid, in the free state or at least partially neutralized, in or for the manufacture of a cosmetic or dermatological composition for combating cutaneous ageing, this acid corresponding to the following formula (I): 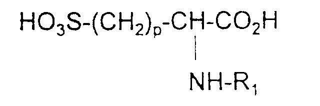 with p having the value 1 or 2 and R₁ representing a hydrogen atom or an acyl residue of structure -COR₂ where R₂ is a linear or branched C₁ to C₁₉ alkyl or alkenyl group which is unsubstituted or substituted by at least one hydroxyl functional group.

3. Use of at least one sulphonic acid, in the free state or at least partially neutralized, in or for the manufacture of a dermatological composition for combating wrinkles and/or fine lines and/or actinic blemishes and/or cutaneous dyschromias and/or scars and/or dermatites, this acid corresponding to the following formula (I): 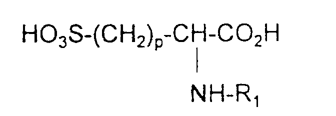 with p having the value 1 or 2 and R₁ representing a hydrogen atom or an acyl residue of structure -COR₂ where R₂ is a linear or branched C₁ to C₁₉ alkyl or alkenyl group which is unsubstituted or substituted by at least one hydroxyl functional group.

4. Use according to one of Claims 1 to 3, characterized in that the sulphonic acid is cysteic acid or homocysteic acid.

5. Use according to one of the preceding claims, characterized in that the sulphonic acid is used in an amount ranging from 0.2 to 20 % and preferably from 0.5 to 5 % by weight with respect to the total weight of the composition.

6. Use according to one of the preceding claims, characterized in that the composition additionally comprises at least one active agent chosen from α- or β-hydroxy acids, α- or β-keto acids and retinoids.

7. Use according to one of the preceding claims, characterized in that the composition additionally comprises at least one active agent chosen from glycolic, lactic, malic, tartaric, citric, 2-hydroxyalkanoic, mandelic, salicylic and 5-(n-octanoyl)salicylic acids.

8. Use according to Claim 6 or 7, characterized in that the active agent is present in an amount ranging from 0.1 to 5 % by weight with respect to the total weight of the composition.

9. Use according to one of the preceding claims, characterized in that the composition additionally contains at least one adjuvant chosen from proteins or protein hydrolysates, amino acids, polyols, urea, sugars and sugar derivatives, vitamins, starch, plant extracts, essential fatty acids, ceramides and essential oils.

10. Use according to one of the preceding claims, characterized in that the composition is an aqueous, oily or aqueous/alcoholic solution, a water-in-oil or oil-in-water emulsion, a microemulsion, an aqueous or anhydrous gel, a serum or a dispersion of vesicles, of microcapsules or of microparticles.

11. Process for the cosmetic treatment of the skin intended for the desquamation of the skin, consisting in applying, to the skin, a composition containing at least one sulphonic acid, in the free state or partially neutralized, of following formula (I): 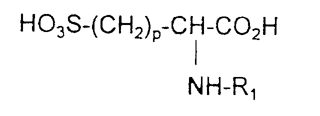 with p having the value 1 or 2 and R₁ representing a hydrogen atom or an acyl residue of structure -COR₂ where R₂ is a linear or branched C₁ to C₁₉ alkyl or alkenyl group which is unsubstituted or substituted by at least one hydroxyl functional group.

12. Process for the cosmetic treatment of ageing of the skin, consisting in applying, to the skin, a composition containing at least one sulphonic acid, in the free state or partially neutralized, of following formula (I): 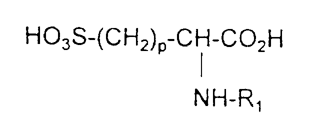 with p having the value 1 or 2 and R₁ representing a hydrogen atom or an acyl residue of structure -COR₂ where R₂ is a linear or branched C₁ to C₁₉ alkyl or alkenyl group which is unsubstituted or substituted by at least one hydroxyl functional group.

## Patentansprüche

1. Verwendung mindestens einer Sulfonsäure im freien Zustand oder wenigstens teilweise neutralisierten Zustand in kosmetischen oder dermatologischen Zusammensetzungen oder zur Herstellung dieser Zusammensetzungen zur Begünstigung der Abschuppung der Haut und/oder Stimulierung der Epidermiserneuerung, wobei die Säure der folgenden Formel (I) entspricht: 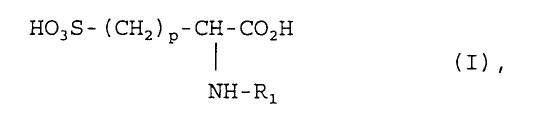 worin
p 1 oder 2 ist und
R₁ ein Wasserstoffatom oder eine Acylgruppe der Formel -COR₂ bedeutet, worin R₂ eine geradkettige oder verzweigte Alkyl- oder Alkenylgruppe mit 1 bis 19 Kohlenstoffatomen ist, die ggf. mit mindestens einer Hydroxygruppe substituiert ist.

2. Verwendung mindestens einer Sulfonsäure im freien Zustand oder wenigstens teilweise neutralisierten Zustand in kosmetischen oder dermatologischen Zusammensetzungen oder zur Herstellung dieser Zusammensetzungen zur Bekämpfung der Hautalterung, wobei die Säure der folgenden Formel (I) entspricht: 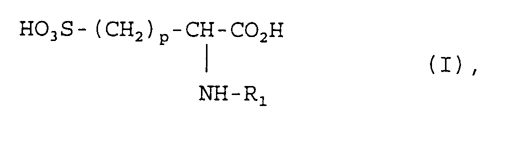 worin
p 1 oder 2 ist und
R₁ ein Wasserstoffatom oder eine Acylgruppe der Formel -COR₂ bedeutet, worin R₂ eine geradkettige oder verzweigte Alkyl- oder Alkenylgruppe mit 1 bis 19 Kohlenstoffatomen ist, die ggf. mit mindestens einer Hydroxygruppe substituiert ist.

3. Verwendung mindestens einer Sulfonsäure im freien Zustand oder wenigstens teilweise neutralisierten Zustand in dermatologischen Zusammensetzungen oder zur Herstellung dieser Zusammensetzungen zur Bekämpfung von Falten und/oder Fältchen und/oder aktinischen Flecken und/oder Hautdyschromien und/oder Narben und/oder Dermatitis, wobei die Säure der folgenden Formel (I) entspricht; 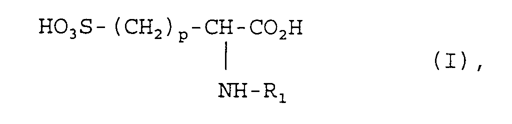 worin
p 1 oder 2 ist und
R₁ ein Wasserstoffatom oder eine Acylgruppe der Formel -COR₂ bedeutet, worin R₂ eine geradkettige oder verzweigte Alkyl- oder Alkenylgruppe mit 1 bis 19 Kohlenstoffatomen ist, die ggf. mit mindestens einer Hydroxygruppe substituiert ist.

4. Verwendung nach einem der Ansprüche 1 bis 3, dadurch gekennzeichnet, daß die Sulfonsäure Cysteinsäure oder Homocysteinsäure ist.

5. Verwendung nach einem der vorhergehenden Ansprüche, dadurch gekennzeichnet, daß die Sulfonsäure in einem Mengenanteil von 0,2 bis 20 Gew.-% und vorzugsweise 0,5 bis 5 Gew.-%, bezogen auf das Gesamtgewicht der Zusammensetzung, verwendet wird.

6. Verwendung nach einem der vorhergehenden Ansprüche, dadurch gekennzeichnet, daß die Zusammensetzung ferner mindestens einen Wirkstoff enthält, der unter den α- oder β-Hydroxysäuren, α- oder β-Ketosäuren und Retinoiden ausgewählt ist.

7. Verwendung nach einem der vorhergehenden Ansprüche, dadurch gekennzeichnet, daß die Zusammensetzung ferner mindestens einen Wirkstoff enthält, der unter Glykolsäure, Milchsäure, Äpfelsäure, Weinsäure, Citronensäure, 2-Hydroxyalkancarbonsäuren, Mandelsäure, Salicylsäure und 5-(n-Octanoyl)-salicylsäure ausgewählt ist.

8. Verwendung nach Anspruch 6 oder 7, dadurch gekennzeichnet, daß der Wirkstoff in einem Mengenanteil von 0,1 bis 5 Gew.-%, bezogen auf das Gesamtgewicht der Zusammensetzung, vorliegt.

9. Verwendung nach einem der vorhergehenden Ansprüche, dadurch gekennzeichnet, daß die Zusammensetzung ferner mindestens einen Zusatzstoff enthält, der unter den Proteinen oder Proteinhydrolysten, Aminosäuren, Polyolen, Harnstoff, Zuckern und Zuckerderivaten, Vitaminen, Stärke, Pflanzenextrakten, essentiellen Fettsäuren, Ceramiden und etherischen Ölen ausgewählt ist.

10. Verwendung nach einem der vorhergehenden Ansprüche, dadurch gekennzeichnet, daß die Zusammensetzung eine wäßrige, ölige oder wäßrig-alkoholische Lösung, eine Wasser-in-Öl-Emulsion oder Öl-in-Wasser-Emulsion, eine Mikroemulsion, ein wäßriges oder wasserfreies Gel, ein Serum, eine Vesikeldispersion, eine Dispersion von Mikrokapseln oder eine Dispersion von Mikropartikeln ist.

11. Verfahren zur kosmetischen Behandlung der Haut zur Abschuppung der Haut, das darin besteht, auf die Haut eine Zusammensetzung aufzutragen, die mindestens eine Sulfonsäure im freien Zustand oder wenigstens teilweise neutralisierten Zustand der folgenden Formel (I) enthält: 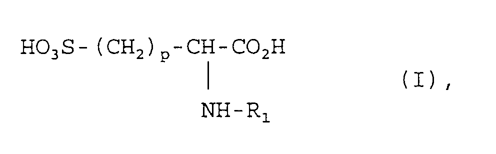 worin
p 1 oder 2 ist und
R₁ ein Wasserstoffatom oder eine Acylgruppe der Formel -COR₂ bedeutet, worin R₂ eine geradkettige oder verzweigte Alkyl- oder Alkenylgruppe mit 1 bis 19 Kohlenstoffatomen ist, die ggf. mit mindestens einer Hydroxygruppe substituiert ist.

12. Verfahren zur kosmetischen Behandlung der Hautalterung, das darin besteht, auf die Haut eine Zusammensetzung aufzutragen, die mindestens eine Sulfonsäure im freien

Zustand oder wenigstens teilweise neutralisierten Zustand der folgenden Formel (I) enthält: 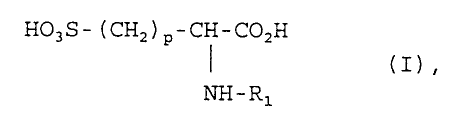 worin
p 1 oder 2 ist und
R₁ ein Wasserstoffatom oder eine Acylgruppe der Formel -COR₂ bedeutet, worin R₂ eine geradkettige oder verzweigte Alkyl- oder Alkenylgruppe mit 1 bis 19 Kohlenstoffatomen ist, die ggf. mit mindestens einer Hydroxygruppe substituiert ist.
